# EUROPEAN PATENT APPLICATION

(11) **EP 3 222 200 A1**
(43) Date of publication of application: **27.09.2017**
(21) Application number: 16855250.3
(22) Date of filing: 26.09.2016
(51) Int. Cl.: A61B 1/04

(54) **ENDOSCOPIC DEVICE**

(30) Priority: 16.10.2015 JP 2015204804
(71) Applicant: Olympus Corporation, Hachioji-shi, Tokyo 192-8507 (JP)
(72) Inventor: IWASAKI, Tomoki, Hachioji-shi Tokyo 192-8507 (JP)
(74) Representative: Gunzelmann, Rainer
(86) International application number: PCT/JP2016/078190
(87) International publication number: WO 2017/064996

(57) **Abstract**

An endoscope apparatus includes: a first display control portion configured to be supplied with patient information that specifies a patient and control display of a patient information image based on the patient information; a second display control portion configured to control display of a warning information image based on warning information regarding a medical action to the patient; and a control portion configured to control the first and second display control portions and cause a display portion to display the patient information image and the warning information image along with a picked-up image obtained by picking up an image of the patient, and configured to independently control the display of the patient information image and the display of the warning information image.

## Description

### Technical Field

The present invention relates to an endoscope apparatus capable of displaying a warning information image based on contraindication information or the like.

### Background Art

Conventionally, endoscopes have been widely adopted in a medical field. In recent years, image quality (definition) improvement of endoscopes has advanced such that tissue inside an abdominal cavity such as a peritoneum structure and a blood stream can be clearly and visually confirmed and reliability of endoscopic surgery and examination has improved.

Incidentally, in examinations and treatment or the like using an endoscope, contraindicated matters that a doctor, a nurse or a technical expert or the like must not perform in order to eliminate medical malpractice exist. For example, xylocaine cannot be used for a patient with a medical history of hypersensitivity to a predetermined anesthetic. In addition, for example, buscopan cannot be administered to a patient with hemorrhagic colitis. Furthermore, for example, depending on a prescribed medicine, biopsy using an endoscope and treatment with an electric knife or the like cannot be performed.

That is, contraindicated matters exist for respective characteristics of patients, situations of medication, and content of treatment and examinations. In addition, sometimes intrinsic precautions such as prohibited treatment that must not be performed for each individual patient are provided in an endoscopic examination or the like. Conventionally, when information on such prohibited treatment or information on the contraindicated matters (referred to as contraindication information, hereinafter) or the like is obtained, the content is indicated to medical staff or the like. For example, for a matter (referred to as a warning matter, hereinafter) that the medical staff must be fully alerted to such as the prohibited treatment and the contraindicated matters, the medical staff writes the content on a sheet of paper or the like and puts up the sheet of paper or the like around an instrument, thereby alerting the medical staff.

However, work of putting up the sheet of paper is troublesome, and a physical space is needed. In addition, normally, an operator is often closely observing a monitor where an endoscopic image is displayed during an examination or the like, and it is possible that operator may overlook the sheet of paper stuck at a position different from the monitor.

In contrast, Japanese Patent Application Laid-Open Publication No. 2011-217854 discloses a technology of providing not only a section to display an endoscopic image but also a section to display basic information (a patient ID, an age, sex or the like) of a patient and operation menu on a display screen of a monitor and providing a comment column where writing can be freely performed in a display section of the basic information, in an endoscope apparatus configured to display the endoscopic image on the monitor. By utilizing the technology in Japanese Patent Application Laid-Open Publication No. 2011-217854 and having a user perform an input operation of information regarding the warning matter (referred to as warning information, hereinafter), it is also possible to display the content of the warning matter in the comment column of the display screen.

Incidentally, in order to display the content of the warning matter on the monitor, the medical staff needs to perform work of inputting a comment while looking at a medical record of the patient for example. However, the work is relatively troublesome since the content needs to be inputted such that the warning matter can be recognized in the comment column. In addition, in order to surely perform an examination or the like, the endoscopic image needs to be displayed on the monitor. However, sometimes it is not needed to display the basic information such as patient information, and the display section of the basic information can be often hidden so that the operator can easily confirm the endoscopic image. However, when the display section of the basic information is hidden, the comment column is also hidden, the content of the warning matter is not displayed, and the medical staff sometimes cannot recognize the content of the warning matter.

An object of the present invention is to provide an endoscope apparatus capable of surely displaying the content of the warning matter without need of troublesome input work.

### Disclosure of Invention

### Means for Solving the Problem

An endoscope apparatus according to one aspect of the present invention includes: a first display control portion configured to be supplied with patient information that specifies a patient and control display of a patient information image based on the patient information; a second display control portion configured to control display of a warning information image based on warning information regarding a medical action to the patient; and a control portion configured to control the first and second display control portions and cause a display portion to display the patient information image and the warning information image along with a picked-up image obtained by picking up an image of the patient, and configured to independently control the display of the patient information image and the display of the warning information image.

### Brief Description of the Drawings

Fig. 1 is a block diagram illustrating an endoscope apparatus relating to a first embodiment of the present invention;
Fig. 2 is an explanatory drawing for describing an entire configuration of a medical system 3 arranged in an operating room;
Fig. 3 is a flowchart for describing an operation of the first embodiment;
Fig. 4 is a flowchart for describing the operation of the first embodiment;
Fig. 5 is an explanatory drawing illustrating one example of display on a display screen of a terminal device 50;
Fig. 6 is an explanatory drawing illustrating one example of display on a display screen of a monitor 60;
Fig. 7 is a block diagram illustrating a second embodiment of the present invention;
Fig. 8 is an explanatory drawing illustrating an in-hospital system;
Fig. 9 is an explanatory drawing for describing content of a database stored in an examination information DB 72a;
Fig. 10 is an explanatory drawing illustrating one example of display on the display screen of the monitor 60 corresponding to Fig. 9; and
Fig. 11 is a flowchart illustrating control of a control portion 40 in the second embodiment.

### Best Mode for Carrying Out the Invention

Hereinafter, embodiments of the present invention will be described in detail with reference to the drawings.

### (First Embodiment)

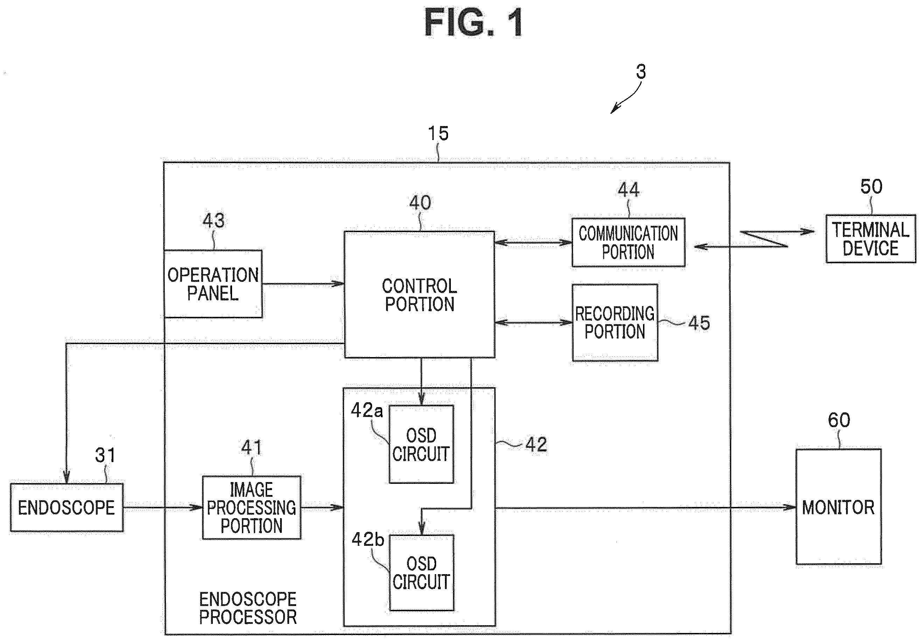

Fig. 1 is a block diagram illustrating an endoscope apparatus relating to a first embodiment of the present invention.

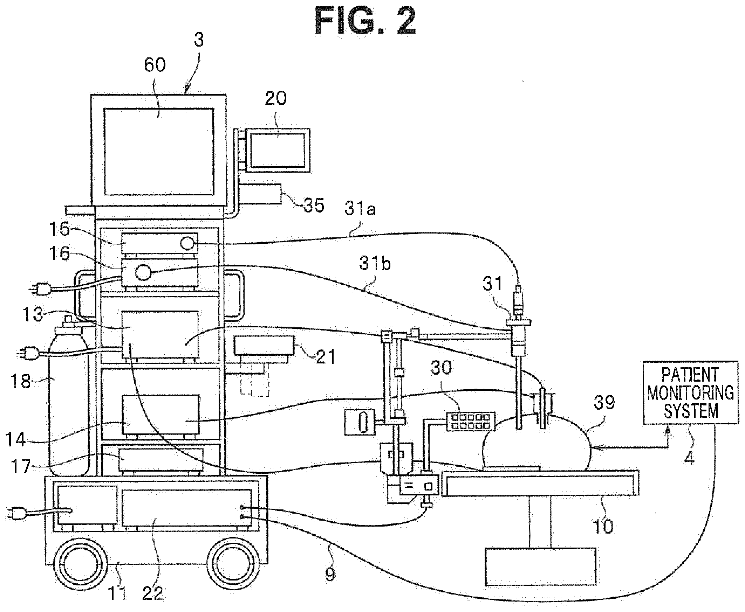

First, an entire configuration of a medical system 3 arranged in an operating room will be described using Fig. 2.

As illustrated in Fig. 2, inside the operating room, a patient bed 10 on which a patient 39 lies down, and the medical system 3 loaded on a cart 11 are arranged. On the cart 11, as medical equipment, devices such as an electrocautery device 13, a pneumoperitoneum device 14, an endoscope processor 15, a light source device 16 and a video recorder 17, and a gas cylinder 18 filled with carbon dioxide are mounted. The endoscope processor 15 is connected to an endoscope 31 through a camera cable 31a.

The light source device 16 is connected to the endoscope 31 through a light guide cable 31b. In addition, on the cart 11, a monitor 60, a central display panel 20, and an operation panel 21 or the like are mounted. The monitor 60 is a TV monitor for example configured to display an endoscopic image or the like.

The central display panel 20 is display means capable of selectively displaying every data during a surgery. The operation panel 21 is configured by a display portion such as a liquid crystal display and a touch sensor for example integrally provided on the display portion, and is a central operation device to be operated by a nurse or the like present in a nonsterile area.

Further, on the cart 11, a system controller 22 which is a central control device is mounted. To the system controller 22, the electrocautery device 13, the pneumoperitoneum device 14, the endoscope processor 15, the light source device 16 and the video recorder 17 described above are connected through a communication line not illustrated in the figure. A headset type microphone not illustrated in the figure can be also connected to the system controller 22, and the system controller 22 can recognize voice inputted from the microphone and control each equipment by the voice of an operator.

In addition, the cart 11 is provided with an RFID (radio frequency identification) terminal 35 capable of wirelessly reading/writing individual ID information of an object by an ID tag embedded in the endoscope 31 or a treatment instrument of the electrocautery device 13 or the like.

In this way, the system controller 22 can centrally control the electrocautery device 13, the pneumoperitoneum device 14, the endoscope processor 15, the light source device 16 and the video recorder 17 loaded on the cart 11. Therefore, in a case that communication is performed between the system controller 22 and the devices, the system controller 22 can display a setting state of a connected device and a setting screen of an operation switch or the like on the liquid crystal display of the operation panel 21 described above. Further, the system controller 22 can perform operation input of setting value change or the like when a desired operation switch is touched and a touch sensor of a predetermined area is operated.

A remote controller 30 is a second central operation device to be operated by a surgeon or the like present in a sterile area, and is capable of operating the other devices with which the communication is established through the system controller 22.

The system controller 22 is connected to a patient monitoring system 4 by a cable 9, and the patient monitoring system 4 can analyze biological information, and display the analysis result at a necessary display device.

In Fig. 1, the endoscope processor 15 is provided with a control portion 40. The control portion 40 may be configured by a processor such as a CPU, or may be operated according to a program stored in a memory not illustrated in the figure and control respective portions inside the endoscope processor 15. The endoscope processor 15 is provided with an operation panel 43, and the operation panel 43 can output an operation signal based on a user operation to the control portion 40. By the user operation to the operation panel 43, input of various kinds of information and various kinds of setting can be performed to the endoscope processor 15.

In addition, the endoscope processor 15 is also provided with a communication portion 44. The communication portion 44 can transmit and receive a signal to/from an outside through a predetermined transmission line. For example, the communication portion 44 can wirelessly transmit and receive the signal, and can transmit and receive information to/from a terminal device 50 by adopting Wifi (wireless fidelity) or Bluetooth (Registered trademark). The terminal device 50 may be configured by a tablet, a PC or a smartphone or the like, and can perform the input of the various kinds of information and the various kinds of setting to the endoscope processor 15.

The endoscope processor 15 can fetch patient information which is the information regarding a patient such as a name of the patient and a patient ID through the operation panel 43 or other input devices not illustrated in the figure, for example. In addition, the endoscope processor 15 can acquire not only the patient information but also the information regarding the examination and status information indicating various kinds of statuses. In following description, the patient information is described as the information also including examination information and various kinds of the status information.

Further, in the present embodiment, to the endoscope processor 15, information (warning information) regarding a warning matter which is a matter that the medical staff must be fully alerted such as prohibited treatment and a contraindicated matter is also inputted. The warning information can be also inputted to the endoscope processor 15 by the operation panel 43, the terminal device 50 or the other input devices not illustrated in the figure.

The control portion 40 of the endoscope processor 15 can acquire the patient information and the warning information, supply and record the patient information and the warning information in a recording portion 45. Note that the recording portion 45 can record not only the patient information and the warning information but also various kinds of setting information, and the control portion 40 sets the respective portions based on the setting information recorded in the recording portion 45. The control portion 40 supplies the acquired patient information to an OSD circuit 42a of an OSD portion 42. In addition, the control portion 40 supplies the acquired warning information to an OSD circuit 42b of the OSD portion 42.

The endoscopic image from the endoscope 31 is supplied to an image processing portion 41 of the endoscope processor 15. The image processing portion 41 executes predetermined image processing on the inputted endoscopic image, and then outputs the endoscopic image to the OSD portion 42. The OSD portion 42 includes the OSD circuit 42a and the OSD circuit 42b. The OSD circuits 42a and 42b are controlled by the control portion 40 and execute onscreen display processing on the inputted endoscopic image.

When the patient information is supplied, the OSD circuit 42a as a first display control portion generates a patient information image indicating content of the patient information by characters such as letters and signs. The OSD circuit 42a sets a display section so as to display the endoscopic image and the patient information image respectively in a predetermined section of a display screen of the monitor 60. The OSD circuit 42a causes the endoscopic image to be displayed in an endoscopic image display section on the display screen, and causes the patient information image to be displayed in a patient information image display section. In addition, for the patient information image, the OSD circuit 42a can switch display and non-display by control of the control portion 40. For example, by a user operating a specific button of the operation panel 43, the patient information image can be displayed or hidden. Further, the OSD circuit 42a can also hide only an image which is a portion of the patient information image.

In the present embodiment, when the warning information is supplied, the OSD circuit 42b as a second display control portion generates a warning information image indicating the content of the warning information by characters such as letters and signs. The OSD circuit 42b sets the display section so as to display the warning information image in the predetermined section of the display screen of the monitor 60. The OSD circuit 42b may display the warning information image in a warning information image display section at a position sufficiently separated from the patient information image. In addition, the OSD circuit 42b may display the warning information image in a non-overlapping area at a position relatively close to the endoscopic image, for example in the warning information image display section above or below the endoscopic image.

In the present embodiment, the OSD circuit 42b may be controlled to display the warning information image at all times. Or, the OSD circuit 42b may be controlled so as to display the warning information image at all times when the endoscopic image is displayed. That is, the OSD circuit 42b may not be able to hide the warning information image by the user operation or the like. In addition, for the warning information image, the OSD circuit 42b sets a color, a font, a letter size, and a background or the like so as to make the warning information image stand out more than the patient information image.

The OSD portion 42 outputs the image generated by the OSD circuits 42a and 42b, that is, the image including the endoscopic image and the patient information image and the warning information image as needed, to the monitor 60. The monitor 60 displays the image from the OSD portion 42 on the display screen.

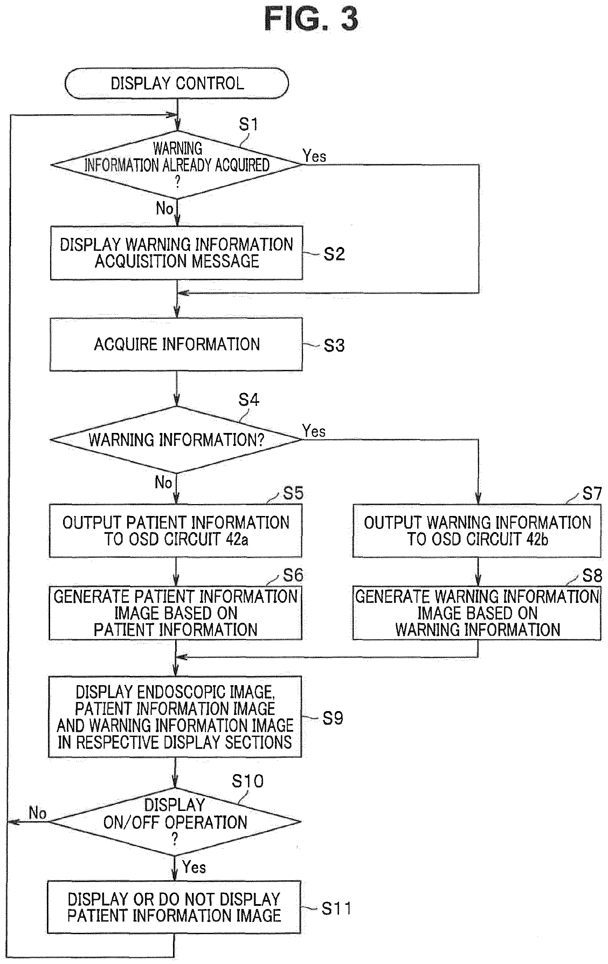

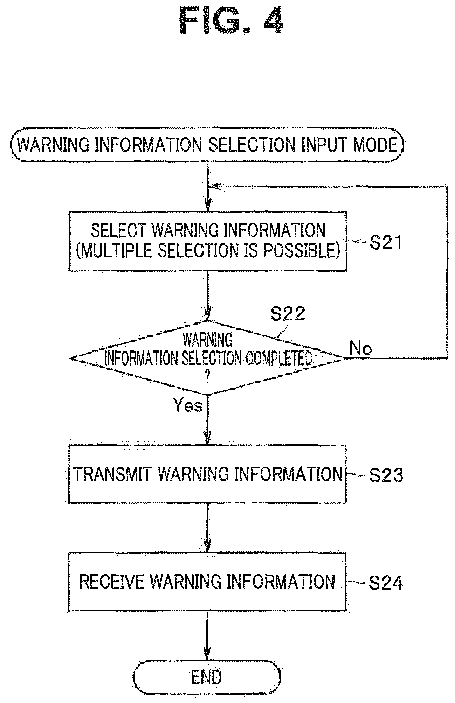

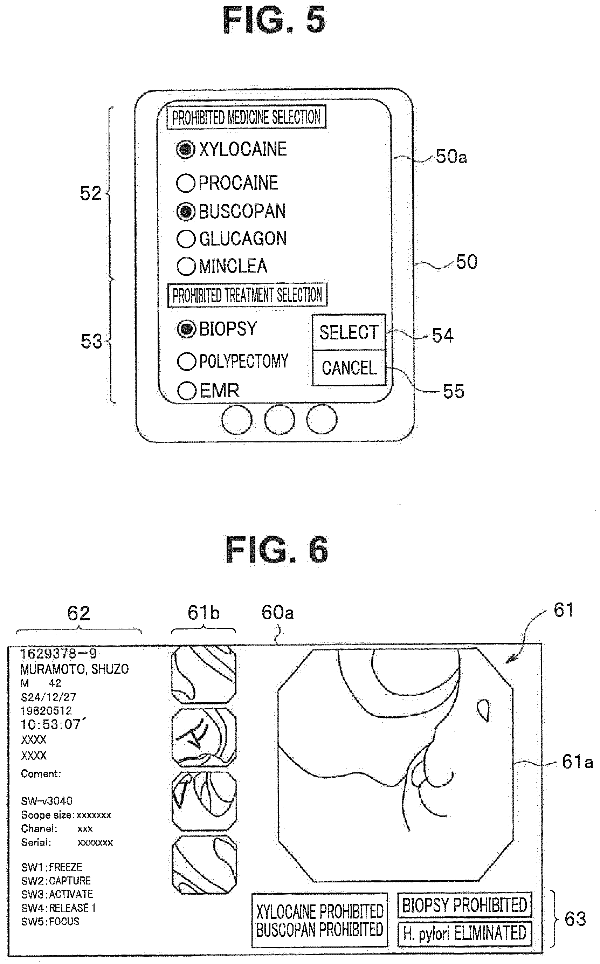

Next, an operation of the embodiment configured in this way will be described with reference to Fig. 3 to Fig. 6. Fig. 3 and Fig. 4 are flowcharts for describing the operation of the first embodiment. In addition, Fig. 5 is an explanatory drawing illustrating one example of display on the display screen of the terminal device 50, and Fig. 6 is an explanatory drawing illustrating one example of the display on the display screen of the monitor 60.

A case of performing an endoscopic examination or the like will be described as an example. Prior to the endoscopic examination, input work of the patient information and the warning information including contraindication information is performed. For example, the medical staff inputs the patient information such as the name of the patient and the patient ID, using the operation panel 43 or the like. In addition, the medical staff performs input work of the warning information including the contraindication information of a prohibited medicine and prohibited treatment or the like using the operation panel 43 or the like. The control portion 40 supplies and records the inputted patient information and warning information in the recording portion 45.

Fig. 4 illustrates a selection input mode of the warning information including the contraindication information, and Fig. 5 illustrates the display on a display screen 50a of the terminal device 50 in the case of inputting the warning information by the terminal device 50. In the terminal device 50, an application configured to input the warning information can be operated, and the display in Fig. 5 is an example of the display by the application.

As illustrated in Fig. 5, on the display screen 50a of the terminal device 50, a prohibited medicine selection area 52 and a prohibited treatment selection area 53 are provided. In the prohibited medicine selection area 52, as the prohibited medicines xylocaine, procaine, buscopan, glucagon and minclea are displayed, and by the user performing a touch operation on the display, the medicines to be specified as the prohibited medicines can be selected. In the example in Fig. 5, by radio button display provided on a left side of the display of each medicine, it is indicated that xylocaine and buscopan are specified.

In addition, in the prohibited treatment selection area 53, as the prohibited treatment, biopsy, polypectomy and EMR (abbreviation for endoscopic mucosal resection) are displayed, and by the user performing the touch operation on the display, the treatment to be specified as the prohibited treatment can be selected. In the example in Fig. 5, by the radio button display provided on the left side of the display of each prohibited treatment, it is indicated that the biopsy is specified.

On the display screen 50a, select button display 54 and cancel button display 55 are also displayed. For example, by the user performing the touch operation on the select button display 54, the terminal device 50 determines the selected prohibited medicine and prohibited treatment and transmits the warning information including the information to the endoscope processor 15. In addition, for example, by the user performing the touch operation on the cancel button display 55, the terminal device 50 can cancel the selected prohibited medicine and prohibited treatment.

The medical staff or the like selects the warning information to be specified in a target medical action, in step S21 in Fig. 4. Completion of the selection is determined by the user performing the touch operation on the select button display 54 (step S22). When the touch operation is performed on the select button display 54, the terminal device 50 transmits all the selected warning information to the communication portion 44 of the endoscope processor 15 (step S23). The control portion 40 supplies and records the warning information received through the communication portion 44 in the recording portion 45 in step S24.

Normally, the input work of the patient information and the warning information is performed before the endoscopic examination is started, however, the input work of the information can be also performed after the endoscopic examination is started.

When the endoscopic examination is started, the control portion 40 of the endoscope processor 15 reads the patient information and the warning information obtained by the input work from the recording portion 45 in step S3. Note that, in order to have the required warning information surely recorded in the recording portion 45 at the point of time, the control portion 40 determines whether or not the warning information such as the contraindication information is already acquired in step S1. In the case that the warning information is not acquired, in step S2, message display is displayed to have the user acquire the warning information such as the contraindication information. The message display may be displayed on the display screen of the monitor 60 by the OSD portion 42, may be displayed on the display screen of the central display panel 20, or may be transmitted to the terminal device 50 through the communication portion 44 of the endoscope processor 15 and displayed on the display screen of the terminal device 50.

In addition, at the terminal device 50 or the like, it is possible to display non-selection button display of the warning information, and in the case that an operation to the non-selection button display is performed, to notify the control portion 40 that there is no warning information to be selected upon the medical action. In the case that the warning information is not recorded in the recording portion 45, it is possible that when there is no response of either reception of the selected warning information or reception of the information indicating that there is no warning information to be selected, the control portion 40 cannot advance processing. Thus, it is possible to prevent an error such as forgetting to display the warning information image.

The control portion 40 determines whether the information read from the recording portion 45 is the warning information such as the contraindication information or the patient information, in step S4, outputs the patient information to the OSD circuit 42a (step S5), and outputs the warning information to the OSD circuit 42b (step S7). The OSD circuit 42a generates the patient information image based on the inputted patient information (step S6). In addition, the OSD circuit 42b generates the warning information image based on the inputted warning information (step S8).

On the other hand, the endoscopic image of the patient is outputted from the endoscope 31. The image processing portion 41 executes predetermined image signal processing on the endoscopic image from the endoscope 31, and then outputs the endoscopic image to the OSD portion 42. The OSD circuit 42a generates display data for displaying the endoscopic image from the image processing portion 41 in the endoscopic image display section and displaying the patient information image in the patient information image display section. In addition, the OSD circuit 42b generates the display data for displaying the warning information image in the warning information image display section. The OSD portion 42 outputs the display data for displaying the endoscopic image, the patient information image and the warning information image in the respective display sections to the monitor 60. The monitor 60 displays the image based on the inputted display data on the display screen.

Note that, in the endoscopic image, not only a movie from the endoscope 31 but also a still image at predetermined photographing timing may be also included. For example, the OSD circuit 42a may display the endoscopic image of the movie in a small section of a sufficiently big size in the endoscopic image display section, and display the endoscopic image of the still image in the small section of a relatively small size in the endoscopic image display section.

Fig. 6 illustrates a display example on a display screen 60a of the monitor 60 in this case. As illustrated in Fig. 6, a patient information image display section 62 is provided in a left side end portion of the display screen 60a, and an endoscopic image display section 61 is provided in a relatively wide area on a right side of the patient information image display section 62. The endoscopic image display section 61 is provided with a small section 61b to display the still image on a left end, and is provided with a small section 61a to display the endoscopic image of the movie in a relatively large area excluding the small section 61b.

In the present embodiment, below the section 61a to display the endoscopic image of the movie, a warning information image display section 63 to display the warning information image is provided.

The endoscopic image being photographed at present is displayed in the section 61 a, and four endoscopic still images photographed at predetermined timing are reduced and displayed in the small section 61b. In addition, in the section 62, the patient information image by letters is displayed. Furthermore, in the example in Fig. 6, by the warning information image displayed in the section 63, it is indicated that xylocaine and buscopan are specified as prohibited medicines, the prohibited treatment of biopsy prohibition is specified, and Helicobacter pylori is already eliminated.

By the warning information image, the medical staff or the like can recognize that, in the medical action, xylocaine and buscopan must not be used, the biopsy must not be performed, and a state of stomach walls is affected by elimination of the Helicobacter pylori and is changed. Since a back ground color, a type of a font, a color and a size or the like are set to be conspicuous for the warning information image, the medical staff or the like can easily confirm the content of the warning information.

The control portion 40 determines whether or not an on (display)/off (non-display) operation of the display of image display is performed in step S10. An operator performing the endoscopic examination sometimes feels that the patient information image displayed on the display screen 60a of the monitor 60 is obstructing a view. In consideration of such a case, the operation panel 43 or the like is sometimes provided with an operation portion for switching the display and the non-display of the patient information image. When it is detected that the operation portion is operated, the control portion 40 displays or hides the patient information image according to the operation in step S11. That is, the OSD circuit 42a does not display the patient information image in the patient information image display section 62 on the display screen 60a, according to the user operation. Note that, depending on the setting, only a portion of the patient information image may be hidden or displayed. Further, the control portion 40 may be able to hide at least one of the endoscopic still image and the endoscopic movie in the endoscopic image display section.

In the present embodiment, the operation portion for hiding the warning information image is not provided. That is, the control portion 40 cannot hide the warning information image. Thus, the medical staff or the like does not erroneously hide the warning information image, the warning information image can be surely displayed, and the medical staff or the like can surely recognize the content of the warning information.

Note that, in the case of executing a safety measure such as displaying a confirmation message for a plurality of times, the warning information image may be hidden based on the user operation. Even in the case, the control portion 40 may perform control so as to continuously display the warning information image during the endoscopic examination, during the display of the monitor, or in the case that the endoscopic image is displayed or the like.

In this way, in the present embodiment, since the display section of the patient information image and the display section of the warning information image are separately set and the display of each display section can be independently controlled, even in the case that the patient information image is hidden, the warning information image can be continuously displayed. Further, it can be prohibited to hide the warning information image, and the medical staff or the like can surely recognize the warning information.

### (Second Embodiment)

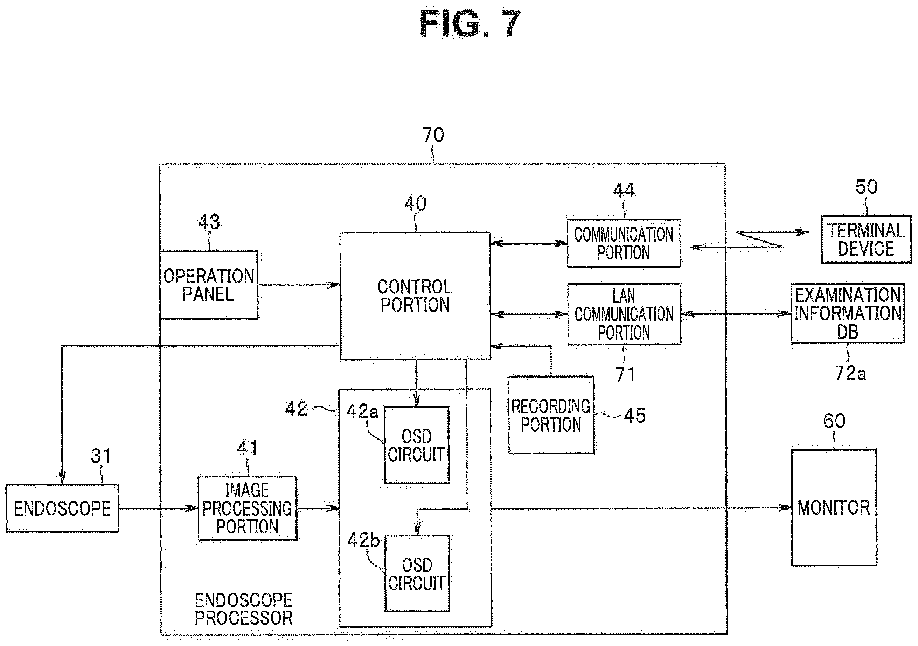

Fig. 7 is a block diagram illustrating a second embodiment of the present invention. In Fig. 7, same signs are attached to same components as the components in Fig. 1, and description is omitted. In the first embodiment, the patient information and the warning information are acquired by manual input by the user. In contrast, in the present embodiment, the patient information and the warning information are automatically acquired from a database.

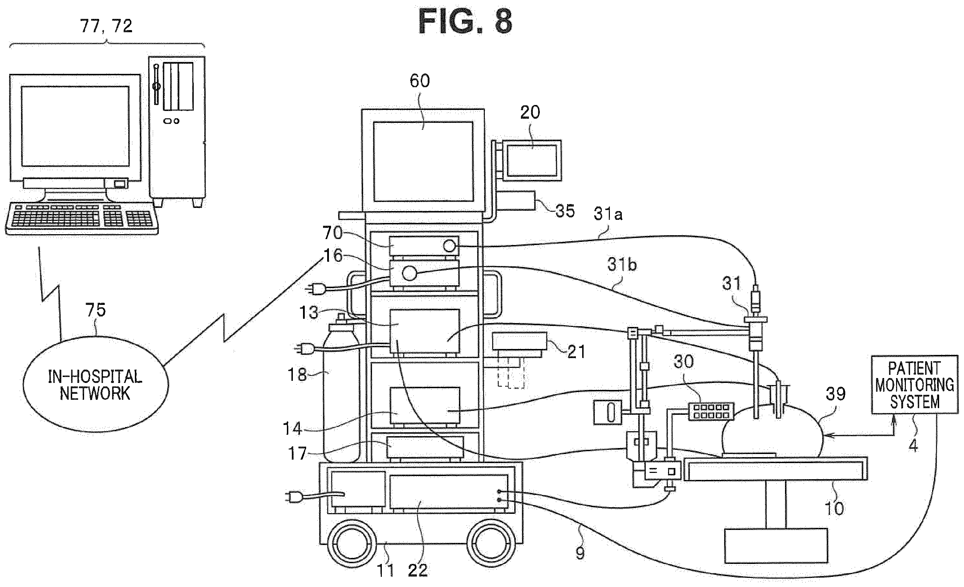

Fig. 8 is an explanatory drawing illustrating an in-hospital system. In Fig. 8, the same signs are attached to the same components as the components in Fig. 2, and the description is omitted. In a hospital, an in-hospital network 75 is configured. On the in-hospital network 75, a computer system (referred to as PC, hereinafter) 77 is provided, and an examination information database (DB) system 72 is constructed by the PC 77. In addition, an examination information DB 72a in Fig. 7 is configured by the PC 77.

In Fig. 7, an endoscope processor 70 includes a LAN communication portion 71. The LAN communication portion 71 can transmit and receive the information to/from the examination information DB 72a through the in-hospital network 75. The LAN communication portion 71 is controlled by the control portion 40, acquires the information stored in the examination information DB 72a, and supplies the information to the control portion 40. The examination information DB system 72 configured by the PC 77 includes the examination information DB 72a in which the patient information and the warning information are registered for each patient, and the examination information DB system 72 can read the information in the examination information DB 72a and transfer the information to the control portion 40.

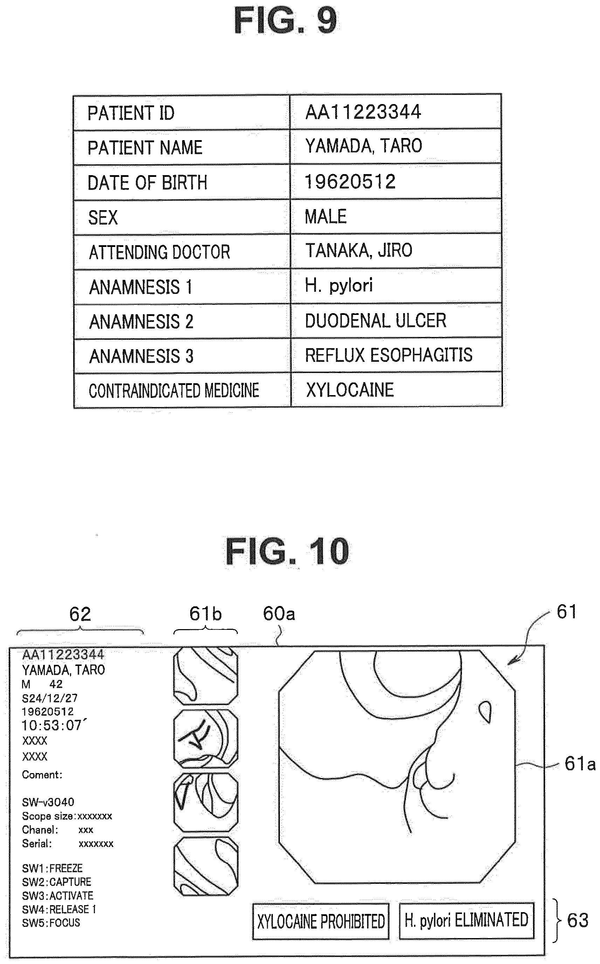

Fig. 9 is an explanatory drawing for describing the content of the database stored in the examination information DB 72a, and illustrates one example of the information registered for one patient. As illustrated in Fig. 9, in the examination information DB 72a, for each patient, the information of a patient ID, a patient name, a date of birth, sex, an attending doctor, anamnesis 1-3, and the contraindicated medicine is registered. Note that, while Fig. 9 illustrates the example that three kinds are registered as the anamnesis and xylocaine is registered as the contraindicated medicine, it is clear that registration information such as the kind of the anamnesis, the contraindicated medicine and the contraindicated treatment is different depending on the patient.

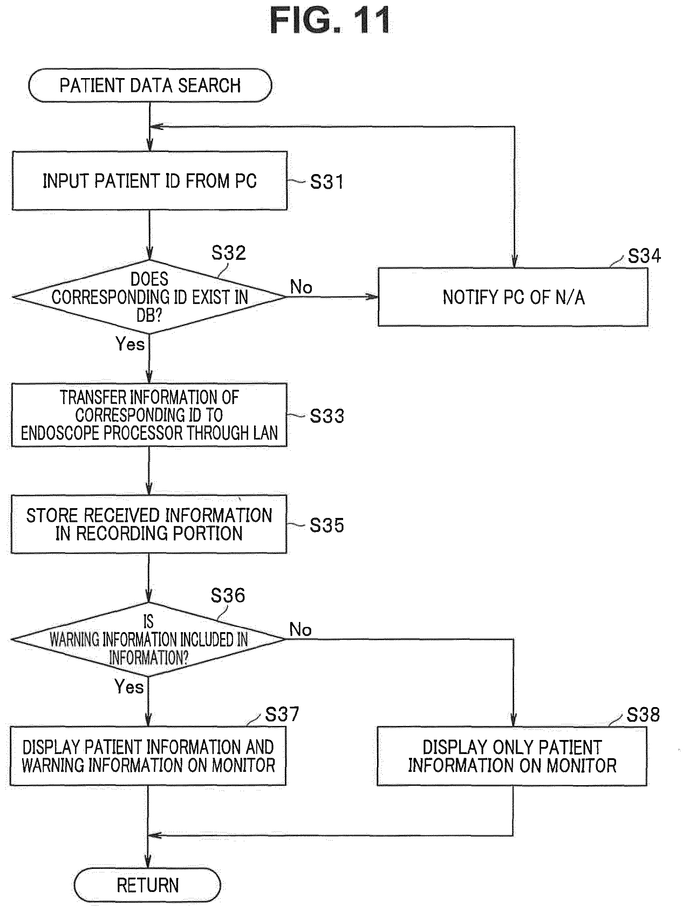

Next, the operation of the embodiment configured in this way will be described with reference to Fig. 10 and Fig. 11. Fig. 10 is an explanatory drawing illustrating one example of the display on the display screen of the monitor 60 corresponding to Fig. 9, and Fig. 11 is a flowchart illustrating control of the control portion 40 in the second embodiment.

Prior to the endoscopic examination or the like, acquisition work of the patient information and the warning information is performed. That is, the medical staff or the like operates the PC 77, inputs the patient ID, and searches the examination information DB 72a, in step S31 in Fig. 11. The examination information DB system 72 determines whether or not registration of a corresponding patient ID exists in the examination information DB 72a (step S32). In the case that the corresponding patient ID is not registered, the examination information DB system 72 notifies the PC 77 of that effect (step S34). In the case that the corresponding patient ID is registered, the examination information DB system 72 reads the information of the corresponding patient ID from the examination information DB 72a, and transmits the information to the endoscope processor 70 (step S33). The LAN communication portion 71 of the endoscope processor 70 transfers the information read from the examination information DB 72a to the control portion 40. The control portion 40 supplies and records the transferred information in the recording portion 45 (step S35).

When the endoscopic examination is started, the control portion 40 reads the information from the recording portion 45 and determines presence/absence of the warning information (step S36). The control portion 40 controls the OSD portion 42 so as to display the patient information and the warning information on the monitor 60 in the case that the warning information is present (step S37), and controls the OSD portion 42 so as to display only the patient information on the monitor 60 in the case that the warning information is not present (step S38).

That is, also in the present embodiment, the patient information is outputted to the OSD circuit 42a, and the warning information is outputted to the OSD circuit 42b.

Fig. 10 illustrates the display example in this case, and according to the information in Fig. 9, the warning information image indicating that xylocaine is the contraindicated medicine is displayed in the warning information image display section 63. In addition, in the section 63, according to the information of the anamnesis 1 in Fig. 9, the warning information image indicating that the Helicobacter pylori is already eliminated is also displayed.

The other operations are similar to the operations in the first embodiment.

Incidentally, in the example in Fig. 9, the example that the information of the contraindicated medicine which is the warning information is recorded is illustrated. However, even in the case that the warning information is not registered in the examination information DB 72a, the warning information such as the contraindicated medicine can be sometimes acquired from the anamnesis or everyday medicines for example. For example, a table describing a relation between the anamnesis and the everyday medicines and the corresponding warning information may be prepared in the recording portion 45 of the endoscope processor 70, and the warning information may be generated based on the read anamnesis and everyday medicine in the control portion 40.

For example, it is assumed that hypertension is registered as the anamnesis in the examination information DB 72a. As a contraindicated medicine for hypertension, glucagon is known. By the control portion 40 referring to the table describing such correspondence between the anamnesis and the contraindicated medicine (warning information), even in the case that the warning information is not registered in the examination information DB 72a, the control portion 40 can generate the warning information from the anamnesis.

In this way, also in the present embodiment, an effect similar to the effect of the first embodiment can be obtained. In addition, in the present embodiment, the patient information and the warning information are acquired using the registration information of the examination information DB 72a, and it is a merit that the input work by the user for the endoscope processor to acquire the information can be extremely simplified.

In the respective embodiments described above, the example that the endoscopic image display section, the patient information image display section and the warning information image display section are provided and the display of the respective display sections can be independently controlled is described. That is, in this case, the display and the non-display of the warning information image can be controlled independent of on/off of the display of the endoscopic image and the patient information image. However, the display and the non-display of the warning information image may be sometimes determined in association with the display and the non-display of the endoscopic image. Then, in this case, the display section of the endoscopic image and the patient information image and the display section of the patient information image may be provided and the display is controlled for the respective display sections.

In addition, since the warning information image indicates the contraindicated medicine and the contraindicated treatment or the like of the medical action, it is preferable to display the warning information image over an entire period from start to end of the endoscopic examination or the like. Furthermore, it is sometimes preferable to display the warning information image before the start of the endoscopic examination, it is also sometimes preferable to display the warning information image only in a display period of the endoscopic image, and the display period of the warning information image may be configured to be controlled by the control portion.

In addition, in the respective embodiments described above, the example that the OSD circuit configured to generate the patient information image and the OSD circuit configured to generate the warning information image are provided as different circuits is described, however, by providing only one OSD circuit and controlling the OSD circuit so as to switch processing on the patient information and the warning information by the control portion, it is clear that display control to the display section of the patient information image and the display control to the display section of the warning information image can be made independent.

In addition, in the respective embodiments described above, the example that the OSD circuits are provided inside the endoscope processor is described, however, at least one of the two OSD circuits may be configured to be incorporated inside the monitor, and a command to control the OSD circuit inside the monitor may be transmitted from the control portion of the endoscope processor together with the patient information and the warning information.

Further, in the respective embodiments described above, the example that the display of the warning information image is controlled by the endoscope processor is described, however, the display of the warning information image may be controlled by the monitor alone. For example, the monitor may be provided with a selection switch of the warning information, and the image according to the warning information may be displayed according to the operation of the switch. The warning information image in this case may be superimposed on the endoscopic image and displayed or may be displayed in the display section different from the endoscopic image.

The present invention is not limited as it is to the embodiments, and constituent elements can be modified and embodied without departing from the scope in an implementation phase. In addition, by an appropriate combinations of the plurality of constituent elements disclosed in the embodiments, various inventions can be formed. For example, some constituent elements of the entire constituting elements indicated in the embodiments may be deleted.

The present application is filed with Japanese Patent Application No. 2015-204804 filed in Japan on October 16, 2015 as a base of a claim of priority, and the above-described disclosure content is incorporated by reference in the present description, and claims.

## Claims

1. An endoscope apparatus comprising:
a first display control portion configured to be supplied with patient information that specifies a patient and control display of a patient information image based on the patient information;
a second display control portion configured to control display of a warning information image based on warning information regarding a medical action to the patient; and
a control portion configured to control the first and second display control portions and cause a display portion to display the patient information image and the warning information image along with a picked-up image obtained by picking up an image of the patient, and configured to independently control the display of the patient information image and the display of the warning information image.

2. The endoscope apparatus according to claim 1,
wherein the control portion sets a first display section to display the patient information image and a second display section to display the warning information image as different display sections, and independently controls the display of the first display section and the display of the second display section.

3. The endoscope apparatus according to claim 2,
wherein the second display section is adjacent to and does not overlap with a display section of the picked-up image.

4. The endoscope apparatus according to claim 1,
wherein the control portion independently controls the display of the picked-up image and the display of the patient information image and the display of the warning information image.

5. The endoscope apparatus according to claim 1,
wherein the control portion controls the display of the picked-up image and the display of the warning information image in correspondence.

6. The endoscope apparatus according to claim 1,
wherein at least one of the first and second display control portions is provided in the display portion.

7. The endoscope apparatus according to claim 1,
wherein the warning information includes information on at least one of a contraindicated medicine and contraindicated treatment of the patient.

8. The endoscope apparatus according to claim 1,
wherein the control portion causes the warning information image to be displayed at least during the display of the picked-up image.

9. The endoscope apparatus according to claim 1, comprising an operation portion configured to input the warning information.

10. The endoscope apparatus according to claim 1, comprising:
a remote terminal configured to input the warning information; and
a communication portion configured to perform communication with the remote terminal and receive the warning information.

11. The endoscope apparatus according to claim 1, comprising
a network reception portion configured to receive the patient information and the warning information from a medical information database including the patient information and the warning information.
